(19) Europäisches Patentamt / European Patent Office / Office européen des brevets

(11) **EP 4 574 046 A1**

(12) **EUROPEAN PATENT APPLICATION**

(43) Date of publication:
**25.06.2025 Bulletin 2025/26**

(21) Application number: **24216921.7**

(22) Date of filing: **02.12.2024**

(51) International Patent Classification (IPC):
**A61B 5/374** *(2021.01)*

(52) Cooperative Patent Classification (CPC):
**A61B 5/374**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.12.2023 CN 202311778465**

(71) Applicant: **Kingfar International Inc.**
**Beijing 100085 (CN)**

(72) Inventors:
• ZHAO, Qichao
  **Beijing (CN)**
• YANG, Ran
  **Beijing (CN)**

(74) Representative: **Meyer-Dulheuer MD Legal Patentanwälte PartG mbB**
**Hanauer Landstr. 287-289**
**60314 Frankfurt am Main (DE)**

(54) **ELECTROENCEPHALOGRAM SIGNAL PREPROCESSING METHOD, SYSTEM AND TERMINAL DEVICE**

(57) Provided is an electroencephalogram (EEG) signal preprocessing method, system and terminal device. The method includes: receiving original EEG signals collected by an EEG collection device, data structure of the original EEG signals corresponds to pre-positioned EEG signal channel locations; performing artifact removal and noise removal operations on the collected original EEG signals to obtain pure EEG signals; obtaining event marks of the original EEG signals, dividing the pure EEG signals into multiple segments each containing EEG signals within preset length of time range before and after an event occurs; obtaining, for the segments, the preset analysis indicators to be extracted, performing time-domain analysis to extract time-domain analysis indicators, and performing frequency-domain analysis to extract frequency-domain analysis indicators. The technical solution can improve the processing efficiency of removing noise and artifacts from the original EEG data, thereby improving the quality of EEG data.

FIG. 1

EP 4 574 046 A1

**Description**

**TECHNICAL FIELD**

The present disclosure relates to the field of electroencephalogram data preprocessing technologies, and particularly relates to an electroencephalogram signal preprocessing method, system and terminal device.

**BACKGROUND**

Electroencephalogram (EEG) data mainly refer to EEG waves. EEG waves mainly appear in the temporal and occipital lobes. For example, $\delta$ rhythm can be observed in the deep-sleep people, infants, and patients with severe organic brain disorders, the waveform is a sinusoidal wave of 30-50$\mu$V, and the amplitude decreases along the occipital-parietal-frontal-temporal direction with a significant amplitude modulation phenomenon under normal circumstances. The $\delta$ rhythm is related to visual activities and presents the largest quantity and the highest amplitude when people are awake, quiet and with their eyes closed. When people fall asleep, $\alpha$ rhythm completely disappears. Factors such as the frequency, amplitude, and spatial distribution of the $\alpha$ rhythm are important indicators reflecting the functional state of the brain. With the maturation of the brain or increase in age, the quantity of the $\alpha$ rhythm gradually increases and its frequency gradually rises. The $\alpha$ rhythm tends to be stable in adulthood, and in old age gradually slows down. In the sleep state, the background rhythm in the light sleep stage is 5-6Hz ($\theta$ wave), and the background rhythm in the deep sleep stage is 2-3Hz ($\delta$ wave).

The original EEG data is a finite discrete time series data composed of many sample points. The number of sample points is determined by the sampling rate. For example, if the sampling rate is 1000Hz, then there are 1000 data sample points per second.

Each sample point data represents the amplitude of the EEG waves, which is called voltage value in physics, and the unit is volt (V). Since EEG signals are usually weak, the more commonly used unit is microvolt ($\mu$V).

The process of EEG data preprocessing refers to performing a series of processing on the original EEG data, so as to extract EEG activities from noise for subsequent EEG data analysis.

In the related process of EEG data preprocessing, it is often necessary for professionals to conduct specialized programming processing for the EEG signal preprocessing. There is a lack of an EEG data preprocessing system that can integrally adapt to the preprocessing requirements of EEG data under different circumstances.

**SUMMARY**

In view of this, embodiments of the present disclosure provide an electroencephalogram (EEG) signal preprocessing method, system and terminal device to eliminate or improve one or more defects existing in the related art.

A first aspect of the present disclosure provides an electroencephalogram (EEG) signal preprocessing method, and the method includes the following steps: receiving original EEG signals collected by an EEG collection device, wherein a data structure of the original EEG signals corresponds to pre-positioned EEG signal channel locations, and each channel corresponds to a coordinate location on a scalp of a subject; performing artifact removal operation and noise removal operation on the collected original EEG signals to obtain pure EEG signals; obtaining event marks of the original EEG signals, and dividing the pure EEG signals into multiple segments, wherein each of the multiple segments contains EEG signals within a preset length of time range before and after an event occurs, and each of the event marks refers to one event; and obtaining, for the segments divided from the pure EEG signals, preset analysis indicators to be extracted, respectively performing time-domain analysis to extract time-domain analysis indicators and respectively performing frequency-domain analysis to extract frequency-domain analysis indicators, and taking the extracted time-domain analysis indicators and frequency-domain analysis indicators as output results of the EEG signal preprocessing.

In some embodiments of the present disclosure, for the received original EEG signals collected by the EEG collection device, the method further comprises: deleting original EEG signals from bilateral mastoid point locations on the scalp of the subject and/or from electrooculogram channels.

In some embodiments of the present disclosure, subsequent to the step of performing artifact removal operation and noise removal operation on the collected original EEG signals, the method further comprises: obtaining target features to be analyzed of EEG data, and selecting a reference electrode of the EEG collection device and a reference electrode calculation method based on the target features to be analyzed, wherein the reference electrode calculation method comprises average reference, bilateral mastoid reference and chain reference.

In some embodiments of the present disclosure, the method further comprises: during the process of receiving the original EEG signals, calculating an amount of data of the original EEG signals collected by the EEG collection device received within a unit time, and sending a sampling rate reduction instruction to the EEG collection device when the amount of data exceeds a preset threshold, so as to load pressure of receiving the original EEG signals.

In some embodiments of the present disclosure, the method further comprises: when an abnormal channel with EEG signal quality lower than a preset threshold or with missing EEG signals is detected, selecting EEG signals from a channel at an adjacent location of the abnormal channel to interpolate and generate a new EEG signal, and using the new EEG signal as the EEG signal from the abnormal channel.

In some embodiments of the present disclosure, the method further comprises: screening the segments divided from the pure EEG signals based on a preset threshold, and finding and deleting segments containing artifacts or outliers beyond the preset threshold.

In some embodiments of the present disclosure, the artifact removal operation is based on blind source separation technology, and the performing artifact removal operation on the collected original EEG signals comprises: decomposing the original EEG signals into multiple independent artifacts or neurons based on the blind source separation technology, using independent component analysis (ICA) technology to identify independent variation sources in the EEG signals, and removing the identified artifacts and/or independent variation sources.

In some embodiments of the present disclosure, the noise removal operation is based on canonical correlation analysis technology, the performing noise removal operation on the collected original EEG signals comprises: using autocorrelation in a given time series to describe signal components in the original EEG signals, finding the highest linear correlation and autocorrelation among the original EEG signals based on the canonical correlation analysis technology, and distinguishing and removing noise from EEG signals based on autocorrelation lower than a threshold.

In some embodiments of the present disclosure, the noise removal operation is based on filtering processing and mathematical algorithm to decompose EEG signals and noise, wherein the filtering processing comprises one or more of high-pass filtering, low-pass filtering, band-pass filtering and notch filtering.

In some embodiments of the present disclosure, the artifact removal operation and noise removal operation are based on a deep learning model, the performing artifact removal operation and noise removal operation on the collected original EEG signals comprises: using EEG data containing artifacts and noise as a training set to train the deep learning model, and using the trained deep learning model for automatic detection and automatic removal of artifacts and noise.

In some embodiments of the present disclosure, the performing artifact removal operation and noise removal operation on the collected original EEG signals further comprises: using any one of signal noise separation (SNS) processing, random sample consensus (RANSAC) processing and cross-validation processing to perform artifact removal operation and noise removal operation on the collected original EEG signals.

In some embodiments of the present disclosure, the time-domain analysis indicators comprise one or more of average voltage, peak voltage, peak-to-peak, standard deviation, root mean square and event-related potential, and the frequency-domain analysis indicators comprise one or more of spectral density, frequency band energy, frequency band ratio, spectral peak, spectral entropy, spectral power density and power spectrum.

A second aspect of the present disclosure provides an EEG signal preprocessing system, including a processor and a memory, computer instructions are stored in the memory, and the processor is configured to execute the computer instructions stored in the memory, and when the computer instructions are executed by the processor, the system implements the steps of the method in any of the above embodiments.

A third aspect of the present disclosure provides a terminal device, and the terminal device is configured to implement the steps of the method in any of the above embodiments.

A forth aspect of the present disclosure provides a computer-readable storage medium, on which a computer program is stored, and when the program is executed by a processor, the steps of the method in any of the above embodiments are implemented.

The EEG signal preprocessing method, system and terminal device according to the present disclosure can perform artifact removal and noise removal on the collected EEG signals to obtain relatively pure EEG data, the pure EEG data is divided into multiple segments, and time-domain analysis and frequency-domain analysis are performed on each segment, thereby completing the EEG signal preprocessing. The EEG signal preprocessing method according to the present disclosure can integrally adapt to the preprocessing requirements of EEG data under different circumstances, and can improve the processing efficiency of removing noise and artifacts from the original EEG data, thereby improving the quality of EEG data and retaining the frequency bands and events of interest.

The additional advantages, purposes, and features of the present disclosure will be partially elaborated in the following description, and will partially become obvious to those ordinary skilled in the art after studying the following text, or can be learned according to the practice of the present disclosure. The purposes and other advantages of the present disclosure can be realized and obtained through the structures specifically pointed out in the specification and the accompanying drawings.

Those skilled in the art will understand that the purposes and advantages that can be achieved by the present disclosure are not limited to the above specific descriptions, and the above and other purposes that the present disclosure can achieve will be more clearly understood based on the following detailed description.

**BRIEF** DESCRIPTION OF DRAWINGS

The accompanying drawings described herein are provided to further understand the present disclosure, which constitute a part of the present disclosure, and do not constitute any limitation to the present disclosure. In the drawings:

FIG. 1 shows an electroencephalogram signal preprocessing method according to an embodiment of the present disclosure.
FIG. 2 shows an example diagram of artifact removal operation and noise removal operation according to an embodiment of the present disclosure.

DESCRIPTION OF EMBODIMENTS

To make the purpose, technical solutions and advantages of the present disclosure clearer, a further detailed description of the present disclosure is provided with reference to the following embodiments and drawings. Here, the explanations of the present disclosure are used to explain the present disclosure, but do not limit the present disclosure.

It should also be noted that in order to avoid obscuring the present disclosure due to unnecessary details, only the structures and/or processing steps closely related to the solutions of the present disclosure are shown in the drawings, and other details that are not significant to the present disclosure are omitted.

It should be emphasized that the term "include/comprise" when used in the context refers to the existence of features, elements, steps or components, but does not exclude the existence or addition of one or more other features, elements, steps or components.

It should also be noted that, unless otherwise specified, the term "connection" in the context refers to direct connection, or indicate indirect connection with an intermediate component.

Below, embodiments of the present disclosure will be described with reference to the drawings. In the drawings, the same reference signs represent the same or similar components, or the same or similar steps.

In order to solve the problems existing in the related EEG signal preprocessing methods, the present disclosure provides an EEG signal preprocessing method, system and terminal device, which improves the processing efficiency of removing noise and artifacts from the original EEG data. Electroencephalography (EEG) data, also known as electroencephalogram, is a method of recording brain activities using electrophysiological indicators.

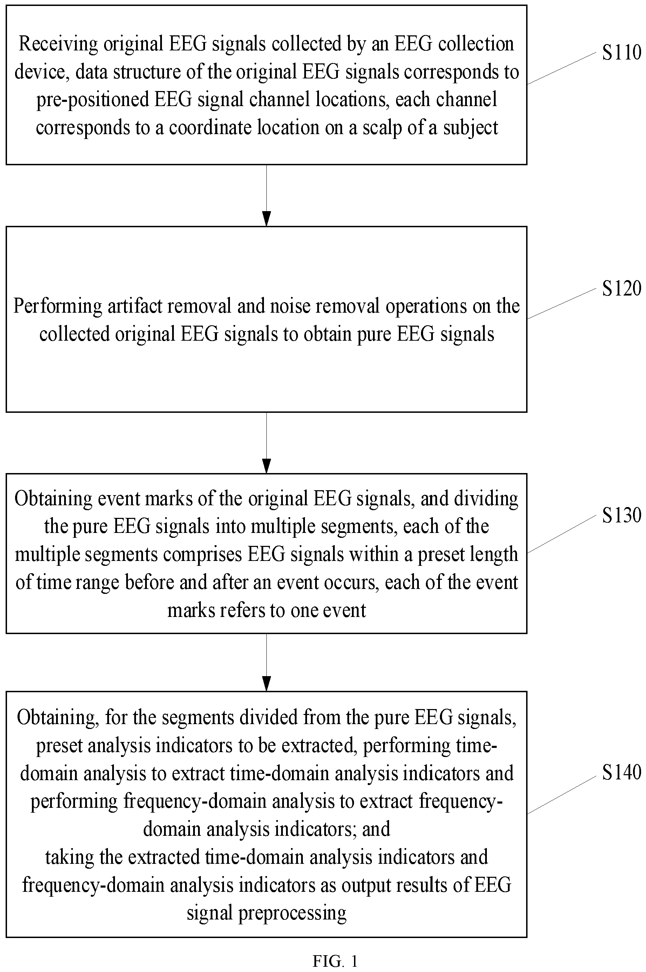

FIG. 1 shows an EEG signal preprocessing method according to an embodiment of the present disclosure. The method includes the following steps:

Step S 110, receiving original EEG signals collected by an EEG collection device, data structure of the original EEG signals corresponds to pre-positioned EEG signal channel locations, and each channel corresponds to a coordinate location on a scalp of a subject.

In some embodiments, positioning channel locations refers to allocating a coordinate position on the scalp for each channel based on the electrode arrangement system, so as to facilitate spatial analysis and visualization. Positioning the EEG signal channel locations is mainly used to determine the locations of the electrodes on the scalp. In neuroelectrophysiological experiments such as electroencephalogram (EEG) or event-related potential (ERP) experiments, multiple electrodes usually need to be placed on the scalp to record the electrical activities of the brain. The locations of these electrodes need to be precisely positioned to accurately measure and record the electrical activities of the brain. The electrode arrangement system of the EEG signals usually adopts the standard electrode placement manner stipulated by the International Federation of Clinical Neurophysiology, namely the 10-20 system. In the 10-20 system, the distance from the midpoint of the frontal pole to the nasion and the distance from the occipital point to the external occipital protuberance each account for 10% of the total length of the connection line, and the remaining points are separated by 20% of the total length of this connection line. Therefore, it is named as the 10-20 system. By positioning the EEG signal channel locations, the specific location of each electrode on the scalp may be determined to ensure the accuracy and reliability of the experimental results. This is of great significance for researching issues in the field of neuroscience such as brain function, cognitive processes, emotions, and attention.

Step S 120: performing artifact removal operation and noise removal operation on the collected original EEG signals to obtain pure EEG signals.

Step S130: obtaining event marks of the original EEG signals, and dividing the pure EEG signals into multiple segments, each of the multiple segments contains EEG signals within a preset length of time range before and after an event occurs, and each of the event marks refers to one event.

In some embodiments, segmentation refers to dividing a long segment of continuous data into segments based on the event marks in the experiment. Each segment contains data within a certain time range before and after an event occurs.

Step S 140: for the segments divided from the pure EEG signals, obtaining preset analysis indicators to be extracted, respectively performing time-domain analysis to extract time-domain analysis indicators and respectively performing frequency-domain analysis to extract frequency-domain analysis indicators, and taking the extracted time-domain analysis indicators and frequency-domain analysis indicators as output results of EEG signal preprocessing.

In embodiments of the present disclosure, the processing efficiency of removing noise and artifacts from the original EEG data can be improved, thereby improving the quality of EEG data and retaining the frequency bands and events of interest.

In an embodiment of the present disclosure, for the received original EEG signals collected by the EEG collection device, the method further includes: deleting original EEG signals from bilateral mastoid point locations on the scalp of the subject and/or from electrooculogram channels. Specifically, according to the purpose and design of the experiment, some unnecessary channels may be removed, such as bilateral mastoid points and electrooculogram channels, etc. combining with other multimodal biological data, artifact data in EEG may be detected and distinguished. For example, combining with electrooculogram and eye movement measurements, blinks and eye movements in EEG signals may be identified. Combining with electrocardiogram signal measurements, electrocardiogram artifacts in EEG may be identified. Combining with acceleration signal measurements, head movement artifacts in EEG may be identified.

In embodiments of the present disclosure, the EEG signals of some unnecessary channels can be deleted, avoiding interference with the feature extraction and use of EEG signals.

In some embodiments of the present disclosure, after the step of performing artifact removal operation and noise removal operation on the collected original EEG signals, the method further includes: obtaining target features to be analyzed of EEG data; and selecting a reference electrode of the EEG collection device and a reference electrode calculation method based on the target features to be analyzed, the reference electrode calculation method includes average reference, bilateral mastoid reference and chain reference. During specific implementation, re-reference refers to choosing an appropriate reference electrode or method based on different analysis targets, such as average reference, bilateral mastoid reference, chain reference, etc., to change the voltage value and spatial distribution of data. There are online reference electrodes in the EEG system, such as the middle location on the top of the head and bilateral earlobes. Reference conversion is a kind of linear conversion, and any activity of the reference electrode will affect the signals of other electrodes. Therefore, the reference electrode should be placed accurately and ensure good contact, otherwise noise will be generated.

In embodiments of the present disclosure, during the process of EEG data processing, re-reference can be performed during offline analysis to reduce the noise data brought by the reference electrode.

In some embodiments of the present disclosure, the method further includes: during the process of receiving the original EEG signals, calculating the amount of data of the original EEG signals collected by the EEG collection device received within a unit time, and sending a sampling rate reduction instruction to the EEG collection device when the amount of data exceeds a preset threshold, thereby reducing load pressure of receiving the original EEG signals. During specific implementation, reducing the sampling rate refers to selecting an appropriate sampling rate based on the Nyquist sampling theorem of signals.

In embodiments of the disclosure, the amount of data and calculation can be reduced, thereby avoiding redundant information brought by excessive sampling.

In some embodiments of the present disclosure, the method further includes: when an abnormal channel with EEG signal quality lower than a preset threshold or with missing EEG signals is detected, selecting EEG signals from a channel at an adjacent location of the abnormal channel to interpolate and generate a new EEG signal, and using the new EEG signal as EEG signal from the abnormal channel. During specific implementation, the channel that needs interpolation refers to a channel with poor data quality or missing data due to poor electrode contact or other reasons.

In embodiments of the present disclosure, it is possible to utilize data of surrounding channels for interpolation supplementation, so as to restore the integrity of data.

In some embodiments of the present disclosure, the method further includes: screening the segments divided from the pure EEG signals based on a preset threshold, and obtaining and deleting segments containing artifacts or outliers beyond the preset threshold. During specific implementation, some segments containing excessive artifacts or outliers may be found and deleted based on some preset thresholds or manual checks to improve data quality. The program can set the threshold, and the abnormal bands can be found, a reasonable filter is selected based on the frequency range of the artifacts, which can effectively reduce artifacts of the original EEG data. For example, the high-pass filtering is set to 0.1Hz, and the low-pass filtering is set to 30Hz. In addition, in order to eliminate interference from the mains supply, notch filtering may be selected to remove interference at 50Hz. At the same time, when interested in a certain signal frequency range, band-pass filtering may be used. For example, if it is interested in the $\alpha$ wave, band-pass filtering may be used to retain the signal frequency band of 8-13Hz for analysis.

In embodiments of the present disclosure, some segments containing excessive artifacts or outliers can be found and deleted to improve data quality.

In some embodiments of the present disclosure, the artifact removal operation is based on blind source separation technology, and the performing artifact removal operation on the collected original EEG signals includes: decomposing the original EEG signals into multiple independent artifacts or neurons based on the blind source separation technology, using Independent Component Analysis (ICA) technology to identify independent variation sources in EEG signals, and removing the identified artifacts and/or independent variation sources. Here, the ICA is a mathematical method.

During specific implementation, the Blind Source Separation (BSS) technology is based on the distinguishable spatiotemporal observation patterns of artifacts and neural signals, effectively decomposing multi-channel EEG data into multiple independent artifacts or neural sources. In EEG analysis, the ICA attempts to identify independent variation sources in EEG data. Each row of the original data matrix represents the situation in which the potential difference between each channel and the reference channel changes over time. After performing ICA decomposition, each row of data represents the situation where the independent components obtained after spatial filtering of the channel data change over time. Therefore, the ICA decomposition provides the temporal and spatial attributes of the independent components. The ICA is an implementation of the BSS. Typical abnormal patterns include: a) energy is concentrated only in the frontal lobe (such as electrooculogram artifacts); b) the topography is discontinuous (such as noise artifacts); and c) the topography is limited to a single electrode point (such as electrode artifacts). The above artifacts are only examples, and the present disclosure is not limited to this.

In embodiments of the present disclosure, mathematical methods may be used to decompose multiple mixed signals into several mutually independent and statistically uncorrelated components to identify and eliminate some artifact components, such as electrooculogram, electromyogram, electrocardiogram, etc.

In some embodiments of the present disclosure, the noise removal operation is based on canonical correlation analysis technology, and the performing noise removal operation on the collected original EEG signals includes: using autocorrelation in a given time series to describe signal components in the original EEG signals, obtaining the highest linear correlation and autocorrelation between the original EEG signals based on the canonical correlation analysis technology, and distinguishing and removing noise from EEG signals based on autocorrelation lower than a threshold.

During specific implementation, Canonical Correlation Analysis (CCA) is a multivariate statistical analysis method used to study the correlation between two groups of variables. It can reveal the intrinsic connection between two groups of variables. In the CCA, the autocorrelation in a given time series is used to describe the signal components in the uncleaned dataset. Autocorrelation describes the similarity between the observed data as a time delay function between them, that is, data points that are close in time usually have strong autocorrelation. The CCA means that the cross-covariance matrix is used to find the highest linear correlation and autocorrelation between datasets. To sum up, this technique can distinguish noise from EEG signals based on relatively low autocorrelation values. Especially for the study of ERP, CCA is considered to create a linear model between the stimuli and the brain responses.

In embodiments of the present disclosure, noise can be distinguished from EEG signals based on relatively low autocorrelation values, thereby filtering out the noise contained in the EEG signals to obtain purer EEG signals.

In some embodiments of the present disclosure, the artifact removal and noise removal operations are based on a deep learning model, and the performing artifact removal and noise removal operations on the collected original EEG signals includes: using EEG data containing artifacts and noise as a training set to train the deep learning model, and using the trained deep learning model for automatic detection and automatic removal of artifacts and noise. During specific implementation, based on the deep learning method, existing datasets are used to train the AI algorithm to automatically detect and reject artifacts in the record (ideally in real time). Mashhadi et al have successfully achieved reliable accuracy to reduce the mean square error between the target signal and the predicted signal.

Furthermore, the performing artifact removal and noise removal operations on the collected original EEG signals further includes: using any one of a Signal - Noise Separation (SNS) method processing, Random Sample Consensus (RANSAC) method processing and Cross-Validation method processing to perform artifact removal operation and noise removal operation on the collected original EEG signals. Through methods such as the SNS method, the RANSAC method, and the Cross-Validation method, the Signal-to-Noise Ratio (SNR) of the EEG signals can be improved to a certain extent.

In some embodiments of the present disclosure, the time-domain analysis indicators include a variety of average voltage, peak voltage, peak-to-peak, standard deviation, root mean square and event-related potential, and the frequency-domain analysis indicators include a variety of spectral density, frequency band energy, frequency band ratio, spectral peak, spectral entropy, spectral power density and power spectrum.

The time-domain analysis indicators of EEG data refer to some parameters used to describe and quantify the characteristics of EEG data in the time domain, such as: (1) average voltage: the arithmetic average of the voltage values of EEG signals within a certain time range, reflecting the overall level of signals; (2) peak voltage: the maximum or minimum voltage value of EEG signals within a certain time range, reflecting the extreme value of signals; (3) peak-to-peak: the difference between the maximum voltage value and the minimum voltage value of EEG signals within a certain time range, reflecting the amplitude range of signals; (4) standard deviation: the average of the square roots of the differences between the voltage values of EEG signals within a certain time range and the average voltage, reflecting the fluctuation degree of signals; (5) root mean square: the square root of the average of the squares of the voltage values of EEG signals within a certain time range, reflecting the effective amplitude of signals; (6) event-related potential (ERP): after aligning EEG signals with an event (such as a stimulus or response), a waveform obtained by averaging all trials at each time point, reflecting the changes in brain electrical activities caused by the event.

The frequency-domain analysis indicators of EEG data refer to some parameters used to describe and quantify the characteristics of EEG data in the frequency domain, such as: (1) spectral density: the energy values of the EEG signals at each frequency, reflecting the frequency distribution and intensity of signals; (2) frequency band energy: the total energy value of EEG signals within a certain frequency band (such as $\delta$, $\theta$, $\alpha$, $\beta$, $\gamma$, etc.), reflecting the activity level of signals in this frequency band; (3) frequency band ratio: the ratio of the energy values of EEG signals within two or more frequency bands, reflecting the relative activity level of signals in different frequency bands; (4) spectral peak: the maximum energy value of EEG signals at a certain frequency, reflecting the dominant frequency and rhythm of signals; (5) spectral entropy: the uncertainty or complexity of the energy values of EEG signals at all frequencies, reflecting the randomness and regularity of signals.

Spectral power density is an indicator that describes the distribution of the power of signals (here referring to EEG signals) in the frequency domain. The physical meaning is the signal power within a unit frequency bandwidth, with the unit watts per hertz (W/Hz). Spectral power density can reveal the energy distribution of signals at different frequencies and plays an important role in analyzing and designing signal processing systems, communication systems, etc.

Power Spectral Density (PSD) is obtained by averaging the data of each channel of over time:

$$result_{ch,i} = \frac{1}{durationIn\,\mathrm{Sec}} \sum_{startTimeIn\,\mathrm{Sec}}^{endTimeIn\,\mathrm{Sec}} psdValues_{ch,j \times segmentLength+i} \; ; (1)$$

The input parameters in the above formula (1) include: (1) psdValues: a two-dimensional array representing PSD values of each channel, each row corresponds to one channel and each column corresponds to a time point; (2) segmentLength: an integer representing the frequency segmentation length of the PSD values; (3) startTimeInSec: an integer representing the starting time point for calculating the average of the PSD values, with the unit of seconds; (4) durationInSec: an integer representing the continuous time period for calculating the average of the PSD values, with the unit of seconds. The output results in the above formula (1) include: result: a two-dimensional array representing the averages of the PSD values of each channel, each row corresponds to one channel and each column corresponds to a frequency segmentation. The specific steps in the above formula (1) include: initializing the result array, to make the number of rows same as the psdValues array and the number of columns equal to the segmentLength.

In each channel (chIndex), the following operation is performed: initializing the result[chIndex] array to have the same length as the psdValues[chIndex] array. For each frequency segmentation (i), the following operation is performed: initializing the variable sum to 0. For each time point (j) from startTimeInSec to endTimeInSec (durationInSec + startTimeInSec), the following operations are performed: adding psdValues[chIndex][j * segmentLength + i] to the variable sum, and dividing the variable sum by durationInSec to obtain result[chIndex][i].

The power spectrum is an indicator used to characterize the energy distribution of signals, and is generally represented by the corresponding power spectral density function. For continuous-time signals, the power spectrum is the Fourier transform of the time-domain signals, representing the amplitudes and phases of different frequency components. For discrete-time signals, the power spectrum is the Fourier transform of the discrete-frequency-domain signals, also representing the amplitudes and phases of different frequency components. The power spectrum estimation belongs to the frequency domain. It means that the total energy of the time series is decomposed into components of different frequencies through the Fourier transform to obtain the power spectral density function of signals.

The power spectrum may be calculated based on the Welch method or the Bartlett method. The Bartlett method is a frequency spectrum estimation method, and the Welch method is also a spectrum estimation method and an improvement of the Bartlett method.

The steps for calculating the power spectrum based on the Welch method are as follows.

First, the data $x(n)$, $n = 0,1,...$ , $N$ -1 with a length of N is divided into L segments, each segment has M data, the data of the i-th segment is expressed as:

$$x_i(n) = x(n + iM - M), 0 \le n \le M, 1 \le i \le L \, ; (2)$$

Then, the window function $w(n)$ is applied to the data of each segment and the periodograms of each segment are calculated. The periodogram of the i-th segment is:

$$I_i(\omega) = \frac{1}{U}\left|\sum_{n=0}^{M-1} x_i(n)w(n)e^{-j\omega n}\right|^2, i=1,2,\ldots,M-1 \qquad ; (3)$$

In the above formula, $U$ is called the normalization factor and the formula is expressed as:

$$U = \frac{1}{M}\sum_{n=0}^{M-1} w^2(n) \qquad ; (4)$$

The periodograms of each segment are approximately regarded as uncorrelated with each other. Finally, the power spectrum estimation is:

$$P_{xx}(e^{j\omega}) = \frac{1}{L}\sum_{i=1}^{L} I_i(\omega) \qquad ; (5)$$

The signals are divided into overlapping segments: the original data is divided into L data segments each with a length of M, and there are D overlapping points between adjacent segments. If D = M/2, the overlap is 50%; if D = 0, the overlap is 0%, which is the same as the Bartlett method.

In the above steps of calculating the power spectrum based on the Welch method, windowing may be applied to each segment: each data segment is multiplied by a window function (in the time domain), the window function usually provides greater weight to the central part of the data and less weight to the edge parts, which means there is a loss of information. In order to reduce this loss, the data segments can have a certain overlap in time (as in the previous step). The periodograms of each segment are calculated: a discrete Fourier transform is performed on each data segment after windowing and then their squared amplitudes are calculated. The periodograms of all segments are averaged, thereby reducing the variance in power spectrum estimation.

An example of algorithm code:

Comment 1: the number of segments and the frequency vector are calculated.

```
int nfft = (int)Math.Pow(2, Math.Ceiling(Math.Log(nperseg, 2)));
```

Comment 2: the number of FFT points is the smallest integer power of 2 that is greater than or equal to nperseg.

```
int nseg = (signal.Length - noverlap) / (nperseg - noverlap);
```

Comment 3: the number of segments is the signal length minus the overlap length divided by the length of each segment minus the overlap length.

```
freq = new double[nfft / 2 + 1];
```

Comment 4: the frequency vector consists of nfft/2+1 points evenly distributed from 0 to half of the sampling frequency.

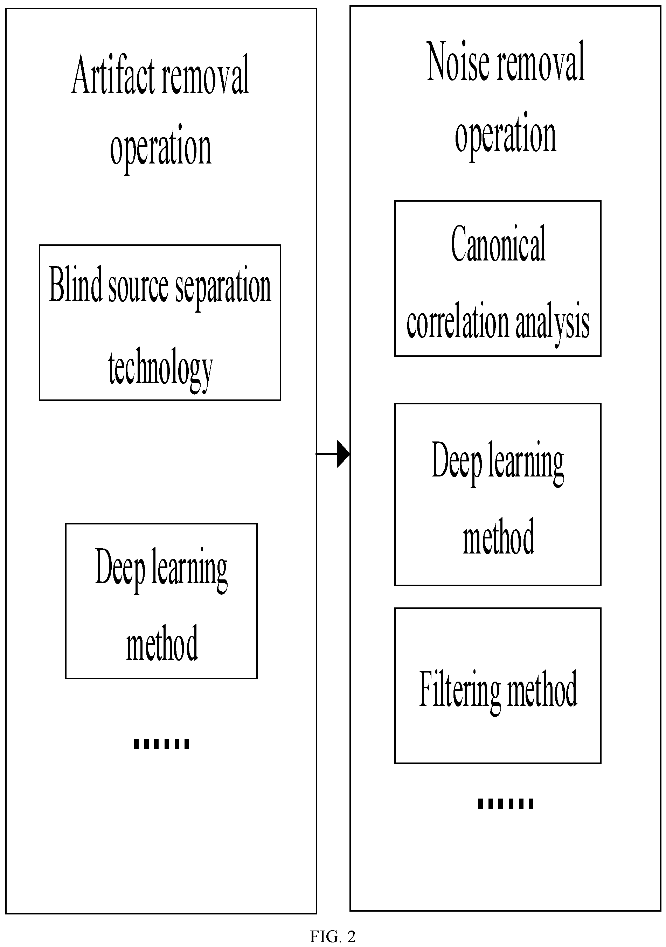

FIG. 2 is an example diagram of artifact removal and noise removal operations according to an embodiment of the present disclosure. After considering artifact removal, there may still be a lot of noise in the collected EEG signals. The signals and noise may be decomposed through post-filtering and mathematical algorithms to improve the signal-to-noise ratio.

It is worth noting that filtering cannot completely filter out the desired frequency band. For example, for a low-pass filtering of 30Hz, it does not mean that all signals outside 30Hz can be completely filtered out. Instead, with 30Hz as the cutoff frequency, signals higher than the cutoff frequency may be gradually attenuated.

In an embodiment of the present disclosure, in a coordinate system, with the horizontal axis representing the frequency and the vertical axis representing the gain coefficient, on the left side of the cutoff frequency f, the initial gain coefficients are all 1, which means that the signals are multiplied by 1 and are completely retained. In a certain range close to the cutoff frequency, the gain coefficient begins to gradually decrease until at the cutoff frequency f, the gain coefficient is exactly 0.5. That is, after low-pass filtering with a cutoff frequency of f, 50% of the data at the f frequency band is filtered out and 50% is retained. Subsequently, the gain coefficient gradually decreases and the signals are gradually attenuated until the gain coefficient approaches 0.

Therefore, in some embodiments of the present disclosure, after performing a low-pass filtering of 30Hz on the EEG signals, a notch filtering of 50Hz may be performed again. This is because performing a low-pass filtering of 30Hz does not completely filter out all signals above 30Hz, and the interference at 50Hz is relatively strong, so it is best to perform another notch filtering of 50Hz.

The introduction to filtering methods is as follows.

(1) High-pass filtering and low-pass filtering: high-pass filtering means that signals higher than a certain frequency can pass through, while signals lower than this frequency may be attenuated and filtered out. On the contrary, low-pass filtering means that signals lower than a certain frequency can pass through, while signals higher than this frequency may be attenuated and filtered out. Therefore, when inputting values, a smaller value should be input for the high-pass filtering, while a larger value should be input for the low-pass filtering.

(2) Band-pass filtering: band-pass filtering means that signals within a certain frequency range can pass through, while signals outside this frequency range may be attenuated and filtered out, which is equivalent to performing high-pass filtering and low-pass filtering simultaneously.

(3) Notch filtering: notch filtering means that signals within a certain frequency range can be attenuated and filtered out, while signals outside this frequency range may be retained. This operation is usually used to remove the interference of mains electricity at 50Hz. In China, the frequency of the mains electricity is 50Hz, so there will be a very strong signal at the frequency band of 50Hz in the collected signals, and notch filtering may be used to remove it.

In the EEG signal preprocessing method, system, and terminal device according to the present disclosure, artifacts and noise from the collected EEG signals can be removed, thereby obtaining relatively pure EEG data. The pure EEG data is divided into multiple segments, and time-domain analysis and frequency-domain analysis are performed on each segment, thereby completing the preprocessing of the EEG signals. The EEG signal preprocessing method according to the present disclosure can integrally adapt to the preprocessing requirements of EEG data under different circumstances, which can improve the processing efficiency of removing noise and artifacts from the original EEG data, thereby improving the quality of EEG data and retaining the frequency bands and events of interest.

Furthermore, the EEG signal preprocessing method and system according to the present disclosure can realize the real-time and uninterrupted preprocessing of EEG signals. The real-time and uninterrupted preprocessing of EEG signals plays an important role in the subsequent artificial intelligence analysis of EEG signals because it can affect the accuracy and reliability of the analysis results. For example, in fields such as EEG-based brain-computer interfaces, diagnosis of neurological diseases, and cognitive function assessment, the selection of EEG data preprocessing methods and parameters will have an impact on the classification or recognition effect.

Corresponding to the above methods, the present disclosure also provides an EEG signal preprocessing system, which includes a processor and a memory. Computer instructions are stored in the memory, and the processor is configured to execute the computer instructions stored in the memory. When the computer instructions are executed by the processor, the system implements the steps of the aforementioned methods.

The present disclosure also provides a terminal device, and the terminal device is configured to implement the steps of the aforementioned methods.

The present disclosure also provides an edge computing terminal device, which may include an intelligent sensor and a programmable logic controller (PLC). The edge computing terminal device is configured to collect data of the steps of any of the aforementioned methods.

An embodiment of the present disclosure also provides a computer-readable storage medium on which a computer program is stored. When the computer program is executed by a processor, the steps of the aforementioned methods are implemented. The computer-readable storage medium may be a tangible storage medium, such as random access memory (RAM), internal memory, read-only memory (ROM), electrically programmable ROM, electrically erasable programmable ROM, registers, floppy disks, hard disks, removable storage disks, CD-ROMs, or any other form of storage medium known in the technical field.

Those of ordinary skill in the art should understand that the exemplary components, systems, and methods described in combination with the disclosed embodiments in the document may be implemented in hardware, software, or a combination of both. Whether it is implemented in hardware or software specifically depends on the specific application and design constraints of the technical solutions. Professional technicians may use different methods to implement the described functions for each specific application, but such implementation should not be considered to be beyond the scope of the present disclosure. When implemented in hardware, it may be, for example, an electronic circuit, an application-specific integrated circuit (ASIC), appropriate firmware, plug-ins, function cards, and so on. When implemented in software, the elements of the present disclosure are programs or code segments used to perform the required tasks. Programs or code segments can be stored in a machine-readable medium or transmitted on a transmission medium or communication link through a data signal carried in a carrier wave.

It should be clear that the present disclosure is not limited to the specific configurations and processing described above and shown in the drawings. For the sake of conciseness, the detailed description of known methods is omitted here. In the above embodiments, several specific steps are described and shown as examples. However, the method process of the present disclosure is not limited to the specific steps described and shown. Those skilled in the art can make various changes, modifications, and additions or change the order of steps after understanding the spirit of the present disclosure.

In the present disclosure, features described and/or illustrated for certain embodiment can be used in the same way or in a similar way in one or more other embodiments, and/or combined with features of other embodiments or replace features of other embodiments. The above is only the preferred embodiments of the present disclosure and is not used to limit the present disclosure. For those skilled in the art, various changes and modifications can be made to the embodiments of the present disclosure. The scope of the present disclosure shall be defined by the appended claims.

**Claims**

1. An electroencephalogram (EEG) signal preprocessing method, comprising:

   receiving original EEG signals collected by an EEG collection device, wherein a data structure of the original EEG signals corresponds to pre-positioned EEG signal channel locations, and each channel corresponds to a coordinate location on a scalp of a subject;
   performing artifact removal operation and noise removal operation on the collected original EEG signals to obtain pure EEG signals;
   obtaining event marks of the original EEG signals, and dividing the pure EEG signals into multiple segments, wherein each of the multiple segments contains EEG signals within a preset length of time range before and after an event occurs, and each of the event marks refers to one event; and
   obtaining, for the segments divided from the pure EEG signals, preset analysis indicators to be extracted, respectively performing time-domain analysis to extract time-domain analysis indicators and respectively performing frequency-domain analysis to extract frequency-domain analysis indicators, and taking the extracted time-domain analysis indicators and frequency-domain analysis indicators as output results of the EEG signal preprocessing.

2. The method according to claim 1, wherein for the received original EEG signals collected by the EEG collection device, the method further comprises:
   deleting original EEG signals from bilateral mastoid point locations on the scalp of the subject and/or from electrooculogram channels.

3. The method according to claim 1, wherein subsequent to the performing artifact removal operation and noise removal operation on the collected original EEG signals, the method further comprises:
   obtaining target features to be analyzed of EEG data, and selecting a reference electrode of the EEG collection device and a reference electrode calculation method based on the target features to be analyzed, wherein the reference electrode calculation method comprises average reference, bilateral mastoid reference and chain reference.

4. The method according to claim 1, further comprising:
   during the process of receiving the original EEG signals, calculating an amount of data of the original EEG signals collected by the EEG collection device received within a unit time, and sending a sampling rate reduction instruction to the EEG collection device when the amount of data exceeds a preset threshold, so as to load pressure of receiving the original EEG signals.

5. The method according to claim 1, further comprising:
   when an abnormal channel with EEG signal quality lower than a preset threshold or with missing EEG signals is detected, selecting EEG signals from a channel at an adjacent location of the abnormal channel to interpolate and generate a new EEG signal, and using the new EEG signal as the EEG signal from the abnormal channel.

6. The method according to claim 1, further comprising:
   screening the segments divided from the pure EEG signals based on a preset threshold, and finding and deleting segments containing artifacts or outliers beyond the preset threshold.

7. The method according to claim 1, wherein the artifact removal operation is based on blind source separation technology, and the performing artifact removal operation on the collected original EEG signals comprises:
decomposing the original EEG signals into multiple independent artifacts or neurons based on the blind source separation technology, using independent component analysis (ICA) technology to identify independent variation sources in the EEG signals, and removing the identified artifacts and/or independent variation sources.

8. The method according to claim 1, wherein the noise removal operation is based on canonical correlation analysis technology, the performing noise removal operation on the collected original EEG signals comprises:
using autocorrelation in a given time series to describe signal components in the original EEG signals, finding the highest linear correlation and autocorrelation among the original EEG signals based on the canonical correlation analysis technology, and distinguishing and removing noise from EEG signals based on autocorrelation lower than a threshold.

9. The method according to claim 1, wherein the noise removal operation is based on filtering processing and mathematical algorithm to decompose EEG signals and noise, wherein the filtering processing comprises one or more of high-pass filtering, low-pass filtering, band-pass filtering and notch filtering, and/or
wherein the time-domain analysis indicators comprise one or more of average voltage, peak voltage, peak-to-peak, standard deviation, root mean square and event-related potential, and the frequency-domain analysis indicators comprise one or more of spectral density, frequency band energy, frequency band ratio, spectral peak, spectral entropy, spectral power density and power spectrum.

10. The method according to claim 1, wherein the artifact removal operation and noise removal operation are based on a deep learning model, the performing artifact removal operation and noise removal operation on the collected original EEG signals comprises:
using EEG data containing artifacts and noise as a training set to train the deep learning model, and using the trained deep learning model for automatic detection and automatic removal of artifacts and noise.

11. The method according to claim 10, wherein the performing artifact removal operation and noise removal operation on the collected original EEG signals further comprises:
using any one of signal noise separation (SNS) processing, random sample consensus (RANSAC) processing and cross-validation processing to perform artifact removal operation and noise removal operation on the collected original EEG signals.

12. An electroencephalogram (EEG) signal preprocessing system, comprising a processor and a memory, wherein computer instructions are stored in the memory, the processor is configured to execute the computer instructions stored in the memory, and when the computer instructions are executed by the processor, the system implements the steps of the method according to any one of claims 1 to 11.

13. A terminal device, wherein the terminal device is configured to implement the steps of the method according to any one of claims 1 to 11.

14. The terminal device according to claim 13, wherein the terminal device comprises:
an edge computing terminal device, comprising an intelligent sensor and a programmable logic controller (PLC), wherein the edge computing terminal device is configured to collect data of the steps of the method implemented by the terminal device.

15. A computer-readable storage medium, on which a computer program is stored, wherein when the program is executed by a processor, steps of the method according to any one of claims 1 to 11 are implemented.

Receiving original EEG signals collected by an EEG collection device, data structure of the original EEG signals corresponds to pre-positioned EEG signal channel locations, each channel corresponds to a coordinate location on a scalp of a subject — S110

Performing artifact removal and noise removal operations on the collected original EEG signals to obtain pure EEG signals — S120

Obtaining event marks of the original EEG signals, and dividing the pure EEG signals into multiple segments, each of the multiple segments comprises EEG signals within a preset length of time range before and after an event occurs, each of the event marks refers to one event — S130

Obtaining, for the segments divided from the pure EEG signals, preset analysis indicators to be extracted, performing time-domain analysis to extract time-domain analysis indicators and performing frequency-domain analysis to extract frequency-domain analysis indicators; and
taking the extracted time-domain analysis indicators and frequency-domain analysis indicators as output results of EEG signal preprocessing — S140

FIG. 1

FIG. 2

Europäisches Patentamt

European Patent Office

Office européen des brevets

## EUROPEAN SEARCH REPORT

**Application Number**

EP 24 21 6921

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/106247 A1 (HE BIN [US] ET AL) 15 April 2021 (2021-04-15) * paragraphs [0019], [0021], [0022], [0024]; figures 1,2 * | 1-15 | INV. A61B5/374 |
| A | US 2019/298212 A1 (SOULET DE BRUGIÈRE QUENTIN [FR] ET AL) 3 October 2019 (2019-10-03) * paragraph [0108] * | 1-15 | |
| A | CN 114 469 135 A (BEIJING BRAIN LAND SCIENCE AND TECH LIMITED COMPANY) 13 May 2022 (2022-05-13) * figure 6, step S201 * | 1-15 | |
| A | MONTAGNA FABIO ET AL: "A machine learning approach for automated wide-range frequency tagging analysis in embedded neuromonitoring systems", METHODS, vol. 129, 22 June 2017 (2017-06-22), pages 96-107, XP085235808, ISSN: 1046-2023, DOI: 10.1016/J.YMETH.2017.06.019 * section 3.2.1. "Pre-processing and artifact removal"; "EEG signals in bad channels..." * * section "Related work, second paragraph, "Conversely, for stimulation frequencies..." * | 1-15 | TECHNICAL FIELDS SEARCHED (IPC) A61B |

The present search report has been drawn up for all claims

| Place of search | Date of completion of the search | Examiner |
|---|---|---|
| The Hague | 15 April 2025 | Knüpling, Moritz |

EPO FORM 1503 03.82 (P04C01)

## ANNEX TO THE EUROPEAN SEARCH REPORT
## ON EUROPEAN PATENT APPLICATION NO.

EP 24 21 6921

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2025

| Patent document cited in search report | Publication date | Patent family member(s) | | Publication date |
|---|---|---|---|---|
| US 2021106247 A1 | 15-04-2021 | NONE | | |
| US 2019298212 A1 | 03-10-2019 | CN | 110248598 A | 17-09-2019 |
| | | EP | 3547911 A1 | 09-10-2019 |
| | | FR | 3059540 A1 | 08-06-2018 |
| | | US | 2019298212 A1 | 03-10-2019 |
| | | WO | 2018104645 A1 | 14-06-2018 |
| CN 114469135 A | 13-05-2022 | NONE | | |

EPO FORM P0459

For more details about this annex : see Official Journal of the European Patent Office, No. 12/82